# EUROPÄISCHE PATENTANMELDUNG

(11) **EP 2 799 036 A1**
(43) Veröffentlichungstag der Anmeldung: **05.11.2014**
(21) Anmeldenummer: 14153794.4
(22) Anmeldetag: 04.02.2014
(51) Int. Cl.: A61F 2/915, A61F 2/958, A61F 2/95

(54) **Intraluminale Endoprothese und Verfahren zur Herstellung desselbe**

(30) Priorität: 02.04.2013 US 201361807349 P
(71) Anmelder: Biotronik AG, 8180 Bülach (CH)
(72) Erfinder: Blaser, Adrian, 8032 Zürich (CH); Moehl, Raimund, 8127 Forch (CH); Kiekbusch, Martin, 18437 Stralsund (DE)
(74) Vertreter: Lindner-Vogt, Karin L.

(57) **Zusammenfassung**

Die vorliegende Erfindung betrifft ein Implantat mit einer durchbrochenen, vorzugsweise hohlzylinderförmigen Grundstruktur zusammengesetzt aus einer Vielzahl von Stegen (8), von denen mindestens jeweils zwei an Knotenelementen (10, 20, 60, 70, 80) miteinander verbunden sind, wobei das Implantat einen expandierten Zustand und einen komprimierten Zustand einnimmt. Um hinsichtlich des Einnehmens des komprimierten Zustands eine größere Sicherheit auch bei komplizierten Bewegungen des Implantats z.B. beim Einführen in ein Gefäß zu gewährleisten, ist an einer Vielzahl von ersten Knotenelementen (10, 60, 70) jeweils mindestens ein erstes Sperrelement (11, 61, 71) und an einer Vielzahl von zweiten, zu jeweils einem ersten Knotenelement (10, 60, 70) benachbarten Knotenelementen (20, 60, 80) jeweils mindestens ein zweites Sperrelement (21, 62, 81) vorgesehen, wobei jeweils ein erstes Sperrelement (11, 61, 71) und ein benachbartes zweites Sperrelement (21, 62, 81) beim Übergang des Implantats von dem expandierten Zustand in den komprimierten Zustand, vorzugsweise beim Crimpen, miteinander verrasten und hierdurch eine Sperre gegen den Übergang zurück in den expandierten Zustand ausbilden, wobei die Sperre gegen eine Kraft in Umfangsrichtung (6) und gegen eine Kraft in radialer Richtung (5) sperrt. Es werden ferner ein System aus einem derartigen Implantat und einem Katheter mit einem Ballon sowie Verfahren zur Herstellung eines solchen Implantats bzw. eines derartigen Systems beschrieben.

## Beschreibung

Die vorliegende Erfindung betrifft ein Implantat, insbesondere eine intraluminale Endoprothese, mit einer durchbrochenen, vorzugsweise hohlzylinderförmigen Grundstruktur zusammengesetzt aus einer Vielzahl von Stegen, die an Knotenelementen miteinander verbunden sind, wobei das Implantat einen expandierten Zustand und einen komprimierten Zustand einnehmen kann, sowie ein Verfahren zur Herstellung eines derartigen Implantats. Die Erfindung betrifft ferner ein System aus einem Katheter mit Ballon und einem solchen Implantat sowie ein Verfahren zur Herstellung eines derartigen Systems.

Medizinische Endoprothesen oder Implantate für die unterschiedlichsten Anwendungen sind in großer Vielfalt aus dem Stand der Technik bekannt. Als Implantate im Sinne der vorliegenden Erfindung sind endovaskuläre Prothesen oder sonstige Endoprothesen, beispielsweise Stents (Gefäß-Stents (vaskulärer Stent, einschließlich des Herzens und Herzklappenstents, z.B. Mitral-Stent, Pulmonalklappenstent), Gallengang-Stents), Endoprothesen zum Verschließen von persitierenden Foramen ovale (PFO), Stent Grafts zur Behandlung von Aneurysmen, Endoprothesen zum Verschließen eines ASD (Vorhofscheidewanddefekt, atrial septal defect) sowie Prothesen im Bereich des Hart- und Weichgewebes zu verstehen.

Heutzutage werden als Implantate besonders häufig Stents eingesetzt, die zur Behandlung von Stenosen (Gefäßverengungen) dienen. Sie weisen eine durchbrochene, vorzugsweise hohlzylinderförmige (rohrförmige) Grundstruktur auf, die an beiden Längsenden offen ist. Die Grundstruktur von herkömmlichen Stents setzt sich dabei häufig aus einzelnen Maschen aus Stegen (Struts) zusammen, welche beispielsweise zick-zack- oder mäanderförmige Strukturen ausbilden. Ein derartiges Implantat wird häufig mittels eines Katheters in das zu behandelnde Gefäß eingesetzt und dient dazu, das Gefäß über einen längeren Zeitraum (Monate bis Jahre) zu stützen. Durch den Einsatz von Stents und gleichzeitiger oder nachfolgender Dilatation können verengte Bereiche in den Gefäßen erweitert werden, so dass ein Lumengewinn resultiert.

Die Stege der Grundstruktur eines solchen Stents laufen in Bereichen zusammen, welche im Folgenden als Knotenelemente bezeichnet werden. Mindestens zwei Stege sind an einem solchen Knotenelement miteinander verbunden. Häufig werden die zick-zack- oder mäanderförmigen Strukturen in Umfangsrichtung mittels sich häufig in Längsrichtung erstreckenden Verbindungsstegen miteinander verbunden. Sowohl Bereiche, in denen die Stege einer in Umfangsrichtung verlaufenden Struktur mit derartigen Verbindungsstegen aneinander stoßen, als auch Bereiche, in denen lediglich Stege der in Umfangsrichtung gebildeten Struktur zusammen laufen, werden hinsichtlich der vorliegenden Erfindung als Knotenelemente bezeichnet.

Stents oder andere Implantate nehmen üblicherweise zwei Zustände an, nämlich einen komprimierten Zustand mit einem kleinen Durchmesser und einen expandierten Zustand mit einem größeren Durchmesser. Im komprimierten Zustand kann das Implantat mittels eines Katheters in das zu stützende Gefäß eingeführt und an der zu behandelnden Stelle positioniert werden. Hierfür wird das Implantat beispielsweise auf den Ballon eines Katheters aufgecrimpt. Am Ort der Behandlung wird das Implantat dann beispielsweise mittels des Ballons des Katheters dilatiert. Auf Grund dieser Durchmesseränderung ist das Implantat hierbei einer mechanischen Belastung ausgesetzt. Weitere mechanische Belastungen des Implantats können während der Herstellung oder bei der Bewegung des Implantats im oder mit dem Gefäß, in das das Implantat eingesetzt ist, auftreten.

Stents werden heutzutage teilweise auch aus sogenannten Formgedächtnislegierungen hergestellt, beispielsweise aus Nitinol, welche eine temperaturabhängige Formumwandlung vollziehen können. Derartige Stents, welche auch als selbst expandierbare Stents bezeichnet werden, können durch Temperaturänderung, insbesondere durch Temperaturerhöhung, von dem komprimierten Zustand (Niedertemperaturphase, Martensit) in den expandierten Zustand (Hochtemperaturphase, Austenit) überführt werden. Eine derartige Temperaturänderung kann durch Aufwärmen auf die Körpertemperatur erreicht werden.

Derzeit auf dem Markt angebotene, selbst expandierbare Systeme aus einem selbst expandierbaren Stent und einem Katheter beinhalten häufig eine schlauchförmige Außenhülle, welche als Expansionssperre über den auf dem Katheter angeordneten Stent geschoben wird und ihn im komprimierten Zustand fixiert. Zur Freigabe des Stents im Körper wird diese Schutzhülle zurückgeführt. Nachteil dieser Außenhülle ist der erhöhte Durchmesser des Systems insbesondere im distalen Bereich aufgrund der Anwesenheit einer zusätzlichen Schicht.

Ferner weist eine solche Außenhülle in Verbindung mit einem gekrümmten Stent eine vergleichsweise große Biegesteifigkeit auf, was die Einführung des Systems in enge Gefäßradien spürbar erschwert oder verhindert. Ein Vergleich der Biegesteifigkeiten im distalen Bereich von Ballon expandierbaren Stentsystemen (ohne Außenhülle) mit selbst expandierenden Stentsystemen zeigt, dass die Biegesteifigkeit für selbst expandierende Systeme etwa zehn Mal so groß ist.

Zudem muss in den meisten Fällen nach der Implantation eines selbst expandierbaren Stents eine Nachdilatation mit einem Ballonkatheter erfolgen, da die Radialkraft eines solchen Stents in der Praxis oft nicht ausreicht, um die Stenose in dem Gefäß vollständig zu öffnen. Die Nachdilatation hat sich in der Praxis jedoch insbesondere aufgrund des zeitlichen Aufwands nicht bewährt, da Kardiologen Ballon expandierende Stentsysteme gewohnt sind, bei denen die Expansion des Stents und die Dilatation des Gefäßes in einem Schritt erfolgt.

Eine Möglichkeit, das gewohnte Vorgehen für Kardiologen zu unterstützen, besteht darin, einen selbst expandierbaren Stent im gecrimpten Zustand ohne die Verwendung einer Außenhülle zu fixieren. Der Stent wird dann nach der Positionierung an der gewünschten Stelle des Körpers mittels eines Ballons dilatiert.

Aus der Druckschrift US 2009/0234429 A1 ist eine selbst expandierende Prothese bekannt, welche eine Vielzahl von Hakenelementen aufweist, welche transversal von jedem in Längsrichtung verlaufenden Steg abstehen. Im expandierten Zustand sind diese Elemente frei am Gitter des Stents angeordnet. Um die Prothese im komprimierten Zustand zu halten, müssen diese Elemente miteinander verhakt werden, wobei hierfür die Hakenelemente verdreht werden müssen. Dies ist nur manuell zu bewerkstelligen, so dass der Übergang in den komprimierten Zustand mit großem Aufwand verbunden ist. Ferner ist nachteilig, dass durch das Verdrehen der Hakenelemente insbesondere die spitzen Enden der Haken von der Außenseite des Stents vorstehen und den Umfang des Systems vergrößern. Beim Einführen des Systems in den Körper und Durchführen durch Gefäß-Engstellen kann sich der Stent bzw. das System an der Gefäßwand verhaken und zu Verletzungen der Gefäße führen. Hierdurch könnte auch eine unerwünscht frühzeitige Öffnung des Stents bewirkt werden.

Aus der Druckschrift EP 1 266 638 B1 ist eine Gefäßstütze bekannt, bei der an dem Gitter mehrere Brücken vorgesehen sind, welche die Stege miteinander verbinden. Jede Brücke greift im komprimierten Zustand mit einer benachbarten Brücke ineinander und jede Brücke ist im expandierten Zustand von der benachbarten Brücke beabstandet. Jede Brücke weist dabei an ihrem vorderen Ende eine Erweiterung auf, welche sich in der Ebene des Umfangs der Gefäßstütze erstreckt. Zum Eingreifen weisen die Stege entsprechende, sich in der Ebene des Umfangs erstreckende Ausnehmungen auf. Nachteilig bei diesen Verbindungselementen in Form von Brücken ist, dass sich diese Verbindungen insbesondere beim Durchlaufen von engen Kurven, d.h. beim Biegen des Stents, voneinander lösen (aufklicken) können, da sie nur in der Umfangsebene bestehen, so dass die Gefahr besteht, dass sich die Gefäßstütze frühzeitig öffnet.

Aus dem Dokument US 2001/0044651 A1 ist außerdem ein Stent bekannt, welcher eine Vielzahl von Gleit- und Arretierungselementen an sogenannten radialen Elementen aufweist, welche mindestens einen Ratschenmechanismus ausbilden. Der Ratschenmechanismus erlaubt das Gleiten der radialen Elemente und hierdurch eine Durchmesseränderung des Stents von dem komprimierten Zustand in den expandierten Zustand, verhindert aber den radialen Recoil aus dem expandierten Zustand. Nachteilig an dieser Lösung ist, dass die radialen Elemente rippenförmig ausgebildet sind, wobei die Rippen in Umfangsrichtung des Stents verlaufen. Im komprimierten Zustand besteht aufgrund der Länge dieser Rippen die Gefahr, dass die Rippen von dem Stent nach außen abstehen und dadurch, wie bei dem oben angegebenen Beispiel aus der Druckschrift US 2009/0234429 A1, die Gefahr von Gefäßverletzungen besteht.

Aus der Druckschrift US 5,733,328 ist ein Stent bekannt, bei dem parallele, fingerförmige, in Umfangsrichtung verlaufende Stege und in Längsrichtung verlaufende Stege vorgesehen sind. Die in Umfangsrichtung verlaufenden Stege weisen nach außen vorstehende konvexe Abschnitte und die in Längsrichtung verlaufenden Stege Einkerbungen auf, welche ineinander einrasten können. Hierdurch kann der Stent bei unterschiedlicher Größe des Durchmessers fixiert werden, wobei der Rastmechanismus lediglich einer Kraft in Umfangsrichtung entgegen wirkt, sich jedoch bei Kräften in radialer Richtung, wie sie während der Einführung eines Stents in den Körper entlang von Gefäßwindungen entstehen, sich hinsichtlich der Arretierung als nicht effektiv erweist. Ferner besteht der zu US 2009/0234429 A1 genannte Nachteil, nämlich die Verletzungsgefahr aufgrund nach außen abstehender Rippen.

Die Aufgabe der vorliegenden Erfindung besteht somit darin, ein Implantat zu schaffen, das eine sichere Arretierung des Implantats im komprimierten Zustand ohne eine Außenhülle bewirkt und das auch während des Einführens in den Körper und beim Durchführen durch enge Gefäßstellen sicher arretiert bleibt. Ferner sollen Verletzungen der Gefäße durch ein Implantat beim Einführen vermieden werden. Die Aufgabe besteht ferner darin, ein entsprechendes System aus Katheter und Implantat zu schaffen sowie ein einfaches und kostengünstiges Herstellungsverfahren für ein derartiges Implantat bzw. ein derartiges System anzugeben.

Die obige Aufgabe wird durch ein Implantat mit den Merkmalen des Anspruchs 1 gelöst.

Insbesondere sind an dem erfindungsgemäßen Implantat an einer Vielzahl von ersten Knotenelementen jeweils mindestens ein erstes Sperrelement und an einer Vielzahl von zweiten, zu jeweils einem ersten Knotenelement in Umfangsrichtung benachbarten Knotenelementen mindestens ein zweites Sperrelement vorgesehen, wobei jeweils ein erstes Sperrelement und ein in Umfangsrichtung benachbartes zweites Sperrelement beim Übergang des Implantats von dem expandierten Zustand in den komprimierten Zustand, vorzugsweise beim Crimpen, miteinander verrasten oder, anders ausgedrückt: ineinander einrasten, und hierdurch eine Sperre gegen den Übergang zurück in den expandierten Zustand ausbilden, wobei die Sperre gegen eine Kraft in Umfangsrichtung und gegen eine Kraft in radialer Richtung sperrt.

Der Vorteil der erfindungsgemäßen Lösung besteht darin, dass die Expansionssperren (erstes Sperrelement und zweites Sperrelement) beim Crimpen miteinander verrasten und dadurch eine selbständige Expansion des Implantats verhindern, auch wenn die Grundstruktur des Stents aus einer Formgedächtnislegierungen hergestellt ist, beispielsweise aus Nitinol besteht, die eine temperaturabhängige Formumwandlung vollzieht. Wird beim Crimpen ein bestimmter Durchmesser des Implantats unterschritten, rasten die Expansionssperren ein und behalten aufgrund Verrastung den Durchmesser des Implantats im komprimierten Zustand bei, auch wenn kleinere Kräfte mit Komponenten in radialer Richtung und/oder in Umfangsrichtung auf das Implantat einwirken, z.B. beim Einführen in den Körper. Die Expansionssperren können durch Expansion mittels eines Ballonkatheters gelöst werden, der höhere Kräfte auf das Implantat aufbringen kann. Anschließend erfolgt eine selbständige Expansion des Implantats.

Hierbei erstrecken sich die Expansionssperren nicht, wie nach dem Stand der Technik, lediglich in Umfangsrichtung, sondern weisen zudem gewissermaßen eine "dritte Dimension" auf. Sie sind, wie weiter unten noch genauer erläutert wird, derart geformt, dass sie auch gegen in radialer Richtung auftretende Kräfte sperren, so dass die Verrastung z.B. beim Durchlaufen von engen Kurven in Gefäßen während des Einführens des Implantats in den Körper nicht geöffnet wird. Hierdurch wird der komprimierte Zustand sicher und von der Bewegung des Implantats unabhängig eingenommen und erst durch die Dilatation mit dem Ballon des Katheters aufgehoben. Die erfindungsgemäße Lösung beinhaltet daher eine größere Anwendungssicherheit.

Während einer Dilatation müssen dabei in vorteilhafter Weise alle über das Implantat verteilten Expansionssperren im Wesentlichen zum gleichen Zeitpunkt, d.h. in einer kleinen Zeitspanne, besonders bevorzugt gleichzeitig, gelöst werden, da bei einem nicht gleichzeitigen, d.h. ungleichmäßigen Lösen die Gefahr einer lokalen Belastungsüberhöhung an einer oder mehrerer Stellen der Grundstruktur besteht, welche zum Bruch des Materials führen kann.

Die Grundstruktur des erfindungsgemäßen Implantats kann eine Formgedächtnislegierung (z.B. Nitinol) oder jedes andere geeignete Material, insbesondere einem metallischen Material, enthalten. Die vorliegende Erfindung ist daher nicht auf selbst expandierbare Implantate, insbesondere selbst expandierbare Stents oder Herzklappenstents, beschränkt. Für selbst expandierbare Stents oder Herzklappenstents bewirkt die Erfindung jedoch, dass auch für diese die in der Medizin etablierte Handhabung mit einem Ballonkatheter angewendet werden kann, nämlich die Expansion des Implantats und die Dilatation des Gefäßes z.B. mittels Ballon an der zu behandelnden Stelle in einem Schritt.

Erfindungsgemäß existieren insbesondere zwei Möglichkeiten, das oben angegebene erste Sperrelement mit einem zweiten Sperrelement zu kombinieren, so dass diese die erfindungsgemäße Verrastung ausbilden. Die Erfindung ist jedoch nicht auf die unten angegebenen Varianten begrenzt.

In einer ersten Variante ist das erste Sperrelement als ein weibliches Element mit einer Hinterschneidung, welches in Umfangsrichtung offen und in radialer Richtung geschlossen ist, beispielsweise als eine zumindest abschnittsweise kugelförmige und/oder abschnittsweise zylinderförmige Ausnehmung, und das zweite Sperrelement als ein komplementäres männliches Element ausgebildet, beispielsweise als ein zumindest abschnittsweise kugelförmiger und/oder zumindest abschnittsweise zylinderförmiger, in Umfangsrichtung von dem Knotenelement abstehender Zapfen. Hierbei verläuft die Längsachse des den zylinderförmigen Zapfen ausbildenden Zylinders parallel zur Längsrichtung des Implantats, welche senkrecht zur Umfangsrichtung und senkrecht zur radialen Richtung verläuft. Das Merkmal, dass das weibliche Element in Umfangsrichtung offen ist, meint, dass eine sich von der Mitte des weiblichen Elements durch die Mitte der

Öffnung erstreckende Mittelline etwa in Umfangsrichtung verläuft. Ferner ist es in vorteilhafter Weise für nicht oder gering elastische Materialien erforderlich, dass der maximale Durchmesser des männlichen Elements in radialer Richtung größer ist als der Durchmesser der Öffnung und kleiner als der maximale innere Durchmesser des weiblichen Elements in radialer Richtung. Bei Verwendung von Werkstoffen mit einer hohen Elastizität, wie z.B. Nitinol, kann auch ein gleicher bis größerer maximaler Durchmesser des männlichen Elements in radialer Richtung im Vergleich zum inneren Durchmesser des weiblichen Elements in radialer Richtung verwendet werden. Durch die angegebenen Größenverhältnisse wird gewährleistet, dass einerseits das männliche Element hinter der Hinterschneidung verrastet und dass andererseits das männliche Element durch das weibliche Element aufgenommen werden kann. Im komprimierten Zustand rastet das männliche Element in das weibliche Element, insbesondere hinter der Hinterschneidung des weiblichen Elements ein.

Alternativ zu der im Querschnitt runden Gestaltung des weiblichen und des männlichen Elements können andere Querschnittsformen, z.B. die Form eines Dreiecks oder Vierecks oder dergl., die jeweils komplementär sind, verwendet werden. Das männliche Element ist vorzugsweise über einen kurzen Hals an dem jeweiligen zweiten Knotenelement befestigt.

Dieses Ausführungsbeispiel beinhaltet eine einfache Lösung dafür, dass das Sperrelement zusammen mit dem zweiten Sperrelement gegen eine Kraft in Umfangsrichtung und gegen eine Kraft in radialer Richtung sperrt. Gegen eine in Umfangsrichtung ausgeübte Kraft sperrt die Hinterschneidung und gegen eine in radialer Richtung ausgeübte Kraft die in Richtung der jeweiligen Kraft liegenden Wände des weiblichen Elements, z.B. die in die Richtung der jeweiligen Kraft liegenden konkaven Wände des weiblichen Elements.

In einer zweiten, ebenfalls einfach realisierbaren Variante rasten zwei gleichartige Sperrelemente ineinander ein. Hierbei ist das erste Sperrelement als ein im Querschnitt U-förmiges Element mit einer ersten Öffnung in eine erste radiale Richtung sowie mit einer an der ersten Öffnung angeordneten Hinterschneidung und das zweite Sperrelement ebenfalls als ein im Querschnitt U-förmiges Element mit einer zweiten Öffnung in eine zweite, zur ersten radialen Richtung entgegengesetzten Richtung sowie mit einer an der zweiten Öffnung angeordneten Hinterschneidung ausgebildet. Das Merkmal, dass die Öffnungen in radialer Richtung liegen, meint, dass die Öffnungen in radiale Richtung zeigen.

Aufgrund der Anordnung der Öffnungen im ersten Sperrelement und im zweiten Sperrelement derart, dass diese jeweils in radialer Richtung ausgerichtet sind, ist es nicht erforderlich, wie beispielsweise bei der Druckschrift US 2009/0234429 A1, Elemente so zu verbiegen, dass sie von der Außenseite des Implantats vorstehen. Sie können beim Crimpen direkt ineinander einrasten und bilden zusammen mit den Hinterschneidungen jeweils eine Sperre sowohl in radialer Richtung als auch in Umfangsrichtung, und zwar in jeweils beide entgegengesetzte Richtungen, aus. Beim Dilatieren durch einen Ballon kann diese Verrastung durch Aufbiegen dieser Sperrelemente auch wieder auf einfachem Wege gelöst werden. Die Verletzungsgefahr beim Durchführen durch Gefäße ist damit minimal.

Von Vorteil ist weiterhin, wenn die Ausdehnung des zweiten Sperrelements in radialer Richtung kleiner ist als die Ausdehnung des jeweils benachbarten Knotenelements in radialer Richtung. Hierdurch wird gewährleistet, dass sich durch das Sperrelement, das nur beispielsweise durch einen kurzen Hals an dem Knotenelement befestigt ist, in radialer Richtung nicht von dem Implantat vorsteht. Die Verletzungsgefahr durch ein derartiges Implantat, das in einem Körperteil angeordnet oder durch ein Körperteil, beispielsweise ein Gefäß, hindurchgeführt wird, ist gegenüber den Lösungen nach dem Stand der Technik verringert.

Die Länge des Halses, an dem ein zweites Sperrelement an dem jeweiligen zweiten Knotenelement befestigt ist, beträgt vorzugsweise weniger als die Hälfte eines Stegdurchmessers. Auch hierdurch wird verhindert, dass die Sperrelemente von dem Implantat nach außen vorstehen und somit die Verletzungsgefahr ebenfalls minimiert.

Vorzugsweise sind an jedem ersten Knotenelement seitlich zwei erste Sperrelemente, d.h. in Umfangsrichtung in beide Richtungen jeweils ein erstes Sperrelement, angeordnet. Entsprechend sind an jedem zweiten Knotenelement seitlich zwei zweite Sperrelemente, d.h. in Umfangsrichtung in beide Richtungen jeweils ein zweites Sperrelement, angeordnet.

Wie unten detaillierter erläutert wird, lässt sich ein erfindungsgemäßes Implantat besonders einfach und kostengünstig basierend auf einem profilierten Rohr herstellen. Entsprechend ist es von Vorteil, dass das erste Sperrelement und das zweite Sperrelement aus jeweils einem (entsprechend geformten) Abschnitt eines sich parallel zur Längsachse des Implantats erstreckenden Profils ausgebildet sind. Ebenfalls ist aus diesem Grund vorteilhaft, dass auch die Stege aus weiteren Abschnitten von sich parallel zur Längsachse des Implantats erstreckenden Profilen ausgebildet sind.

Die obige Aufgabe wird ferner durch ein System aus einem Implantat und einem Katheter mit einem Ballon gelöst, wobei das Implantat, das die oben beschriebenen Merkmale aufweist, auf den Ballon des Katheters aufgecrimpt ist.

Die Vorteile des Implantats wurden bereits oben erläutert und diese Vorteile führen auch zu entsprechenden Vorteilen des angegebenen erfindungsgemäßen Systems.

Die obige Aufgabe wird außerdem durch ein Verfahren zur Herstellung eines Implantats, insbesondere des oben beschriebenen Implantats, gelöst, mit den folgenden Schritten:
- Herstellen eines Ausgangsrohrs, vorzugsweise mittels Gießen, Rapid Prototyping oder Sintern und ggf. Kernlochbohren,
- Umformen des Ausgangsrohrs zu einem Profilrohr mittels Durchdrücken oder Durchziehen und unter Anwendung eines Werkzeugs umfassend eine Matrize und einen Stopfen, vorzugsweise mittels Strangpressen,
- Ausschneiden der Knotenelemente und der Stege aus dem Profilrohr mittels Laserstrahlschneiden, wobei die Laserstrahlen vorzugsweise im Wesentlichen in radialer Richtung verlaufen.

Der Vorteil des erfindungsgemäßen Herstellungsverfahrens besteht darin, dass durch die Profilierung des Ausgangsrohrs mittels Durchdrücken oder Durchziehen bereits die gewünschte Form der Sperrelemente ausgebildet wird. Danach müssen lediglich die Knotenelemente mit den Sperrelementen und die Stege durch Laserstrahlschneiden, vorzugsweise durch sehr einfach zu realisierende radiale Schnitte, bei denen die Lichtquelle innerhalb oder außerhalb des Implantats angeordnet ist, herausgeschnitten werden.

Während des Umformens erfolgt die Profilierung des Rohrs durch die Matrize auf der Außenseite des Rohrs sowie durch den Stopfen auf der Innenseite. Der Stopfen wird daher auch als Dorn bezeichnet. Das Werkzeug ist üblicherweise beheizt und drehbar gelagert.

Alternativ zu dem obigen Verfahren kann das erfindungsgemäße Implantat auch direkt mittels Rapid Prototyping ausgeformt werden. Rapid Prototyping ist ein einfaches und prinzipiell bekanntes Verfahren für die Herstellung komplexer Geometrien, das jedoch nicht für die Anwendung für große Serien gedacht ist.

Als weitere Alternative werden nach Herstellen eines Ausgangsrohrs, vorzugsweise mittels Gießen oder Sintern oder Rapid Prototyping und ggf. Kernlochbohren, zunächst die Knotenelemente und die Stege der Grundstruktur aus dem Ausgangsrohr mittels Laserstrahlschneiden, vorzugsweise durch im Wesentlichen in radialer Richtung verlaufende Schnitte, d.h. einen in radialer Richtung verlaufenden Laserstrahl, ausgeschnitten. Parallel dazu kann das Verschlusssystem hergestellt werden, beispielsweise aus einem Schweißdraht bzw. -band. Das Verschlusssystems bestehend aus einer Vielzahl von ersten Sperrelementen und von zweiten Sperrelementen wird dann abwechselnd an in Umfangsrichtung jeweils benachbarten Knotenelemente, vorzugsweise mittels Kleben oder Schweißen oder Pulverspritzgießen (Powder Injection Molding - PIM) oder Erodieren (z.B. Bohr-, Senk- oder Drahterodieren), angebracht. Dieses Verfahren ist insbesondere dann von Vorteil, wenn an den Knotenelementen des Implantats unterschiedliche Verschlusssysteme angeordnet werden sollen. Alternativ können die Sperrelemente mittels selektivem Laserschmelzen oder Lasersintern hergestellt und mit den Knotenelementen verbunden werden.

Die obige Aufgabe wird ferner durch ein Verfahren zur Herstellung eines Systems aus einem Katheter mit Ballon und einem Implantat gelöst, wobei das Implantat nach einem der oben angegebenen Verfahren hergestellt wird und dann auf dem Ballon des Katheters angeordnet und auf diesen aufgecrimpt wird.

Das erfindungsgemäße Implantat wird nachfolgend in Ausführungsbeispielen anhand von Figuren erläutert. Dabei bilden alle beschriebenen und/oder bildlich dargestellten Merkmale den Gegenstand der Erfindung, auch unabhängig von ihrer Zusammenfassung in den Ansprüchen oder deren Rückbezügen.

Es zeigen schematisch:
- Fig. 1: einen Querschnitt durch ein erstes Ausführungsbeispiel eines erfindungsgemäßen Implantats im Bereich von ersten und zweiten Knotenelementen,
- Fig. 2: einen Querschnitt durch ein Profilrohr, aus dem das erste Ausführungsbeispiel gemäß Fig. 1 hergestellt wird,
- Fig. 3: den Querschnitt gemäß Fig. 2 während des Laserstrahlschneidens,
- Fig. 4: ein erstes Knotenelement mit sich daran anschließenden Stegen des Ausführungsbeispiels gemäß Fig. 1 in einer perspektivischen Ansicht von der Seite,
- Fig. 5: einen Längsschnitt durch ein Werkzeug für das Strangpressen mit einem darin angeordneten Ausgangsrohr,
- Fig. 6: einen Querschnitt durch das Werkzeug gemäß Fig. 5 in einer Prinzipskizze, wobei der Rohrquerschnitt kein Profil aufweist,
- Fig. 7: einen weiteren Querschnitt durch das Werkzeug gemäß Fig. 5,
- Fig. 8: ein Profilrohr in einer perspektivischen Ansicht von der Seite,
- Fig. 9: den Querschnitt gemäß Fig. 7 sowie den Verlauf der Laserstrahlen beim Laserstrahlschneiden,
- Fig. 10a: einen Querschnitt durch ein erfindungsgemäßes Implantat gemäß dem ersten Ausführungsbeispiel nach dem Laserstrahlschneiden,
- Fig. 10b bis 10d: eine Ansicht des erfindungsgemäßen Implantats gemäß dem ersten Ausführungsbeispiel nach dem Laserschneiden im expandierten Zustand in einer perspektivischen Ansicht von der Seite in verschiedenen Ausschnitten,
- Fig. 10e bis 10g: eine Ansicht des erfindungsgemäßen Implantats gemäß dem ersten Ausführungsbeispiel nach dem Laserschneiden im komprimierten Zustand in einer perspektivischen Ansicht von der Seite in verschiedenen Ausschnitten,
- Fig. 11: Querschnitte durch benachbarte Knotenelemente gemäß einem zweiten Ausführungsbeispiel eines erfindungsgemäßen Implantats nach der Herstellung eines entsprechenden Halbzeugs ohne Sperrelemente,
- Fig. 12: die Querschnitte durch benachbarte Knotenelemente gemäß Fig. 13 nach einem weiteren Schritt des Herstellungsverfahrens mit Sperrelementen im expandierten Zustand,
- Fig. 13: die Knotenelemente gemäß Fig. 12 im komprimierten Zustand,
- Fig. 14: einen Querschnitt durch ein erfindungsgemäßes Implantat in einem dritten Ausführungsbeispiel im Bereich der ersten und zweiten Knotenelemente im komprimierten Zustand und
- Fig. 15: das Ausführungsbeispiel gemäß Fig. 14 während des Laserstrahlschneidens eines Profilrohr-Halbzeugs in einem Querschnitt.

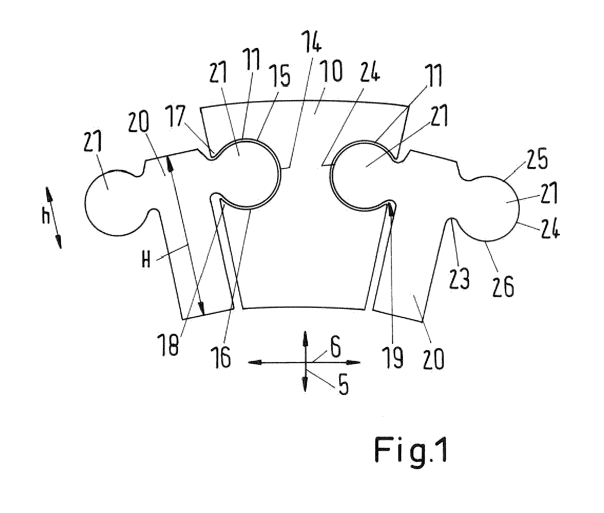

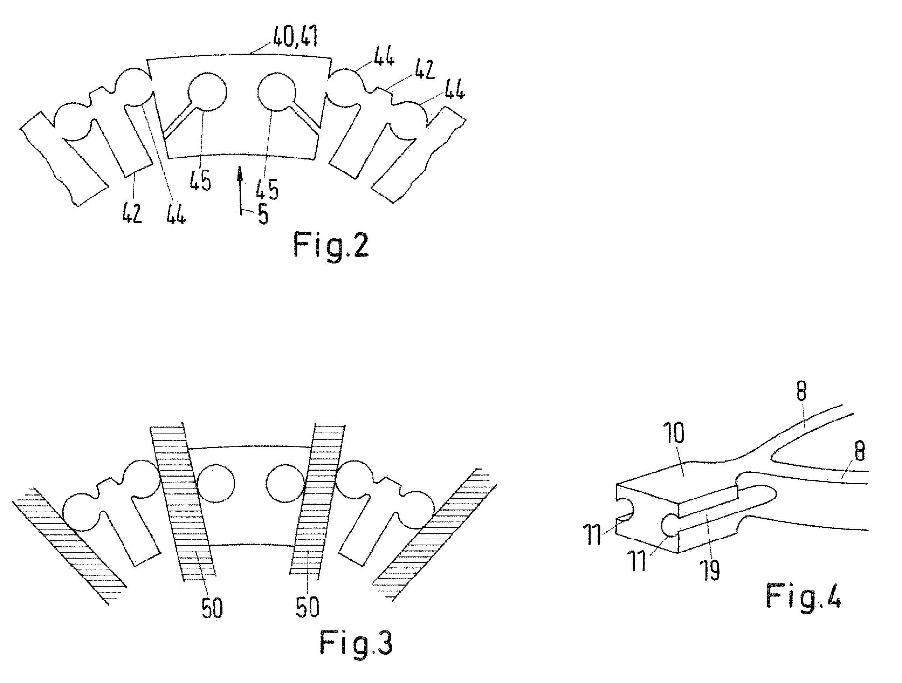

Anhand der Fig. 1 bis 10g wird im Folgenden als ein erstes Ausführungsbeispiel eines erfindungsgemäßen Implantats ein Stent beschrieben, welcher aus einem hohlzylinderförmigen oder rohrförmigen Grundkörper mit einer Vielzahl von Stegen 8 (siehe Fig. 4) zusammengesetzt ist. Die Stege 8 sind in Umfangsrichtung 6 im Wesentlichen zick-zack-förmig angeordnet und verlaufen im Wesentlichen schräg zur Längsachse (Längsrichtung) des Stents. Jeweils mindestens zwei Stege 8 stoßen an ersten Knotenelementen 10 und zweiten Knotenelementen 20 aneinander, wobei ein erstes Knotenelement 10 und ein zweites Knotenelement 20 in Umfangsrichtung 6 abwechselnd vorgesehen sind.

Die Längsrichtung des Stents verläuft senkrecht zu der in Fig. 1 gezeigten Ebene sowie senkrecht zur radialen Richtung 5 und zur Umfangsrichtung 6.

In dem ersten Knotenelement 10 sind seitlich, d.h. in Umfangsrichtung, zwei zylinderförmige Ausnehmungen 11 angeordnet, welche jeweils ein erstes Sperrelement darstellen. In dem in Fig. 1 gezeigten komprimierten Zustand des Stents ist in jeder Ausnehmung 11 ein im Wesentlichen zylinderförmiger Zapfen 21 als zweites Sperrelement angeordnet, welcher über einen Hals 23 an dem zweiten Knotenelement 20 befestigt ist. Der Durchmesser h des Zapfens 21 ist geringfügig kleiner als der Innendurchmesser der Ausnehmung 1l, so dass die Ausnehmung 11 den Zapfen 21 im komprimierten Zustand des Stents, wie in Fig. 1 gezeigt, aufnehmen kann.

Die Ausnehmung 11 weist konkave Wandabschnitte 14, 15 und 16 auf. Entsprechend sind an dem Zapfen 21 an dem dem zweiten Knotenelement 20 gegenüber liegenden Ende ein konvexer Endabschnitt 24 und, jeweils benachbart zu dem Endabschnitt 24 in Richtung des Halses 23, jeweils weitere konvexe Abschnitte 25, 26 vorgesehen.

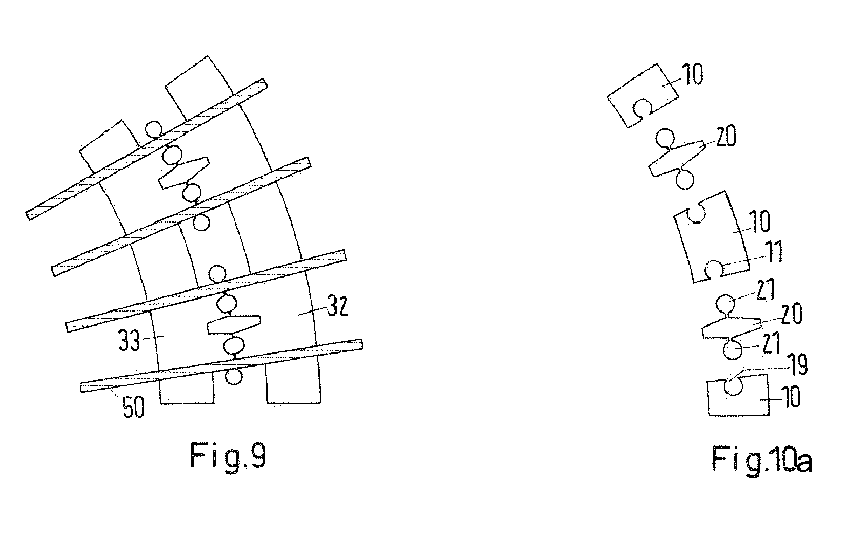

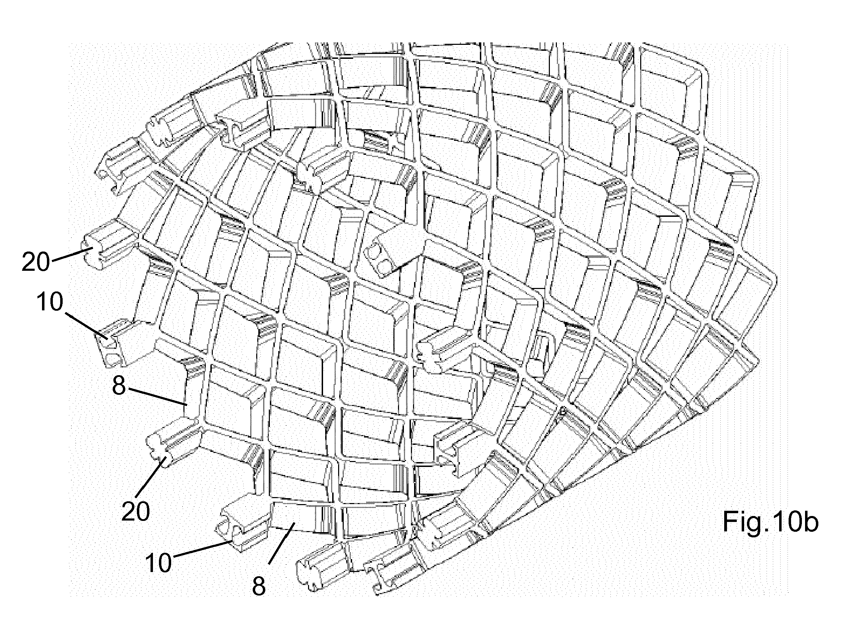

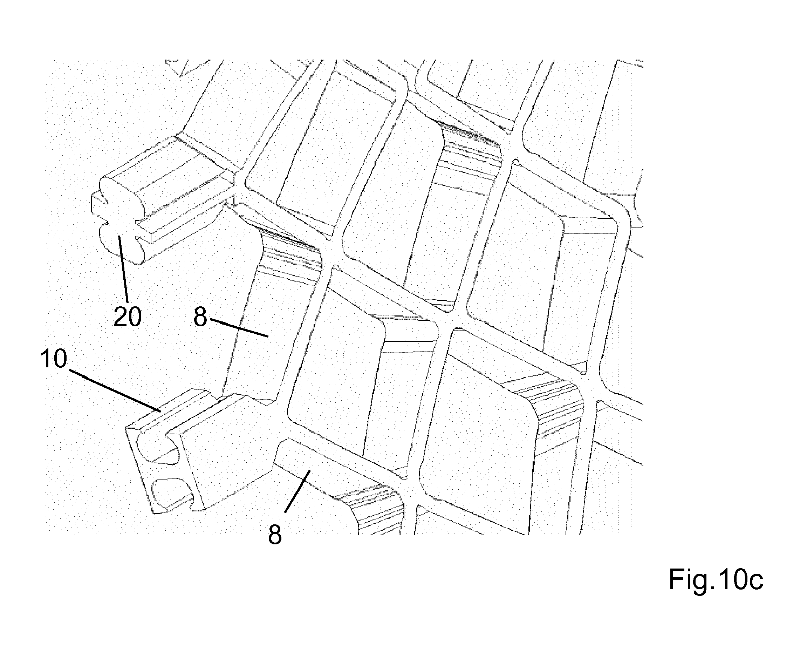

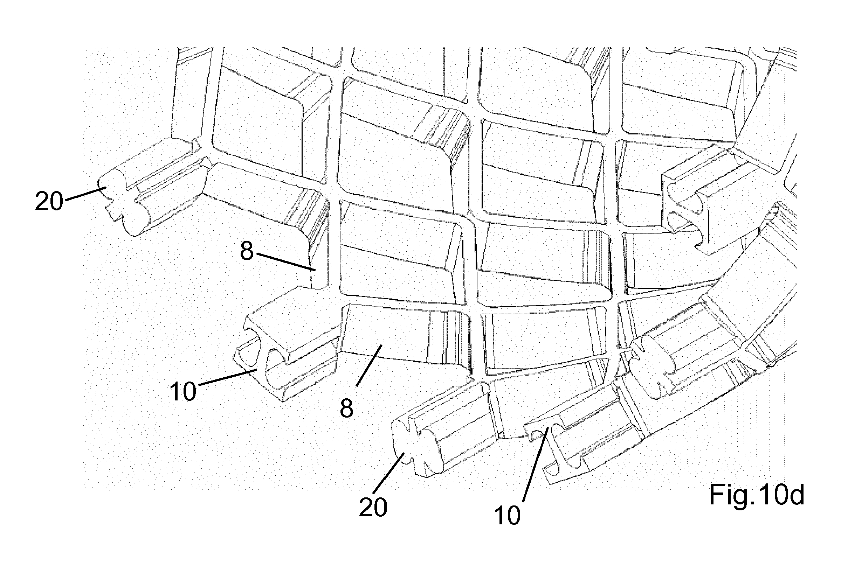

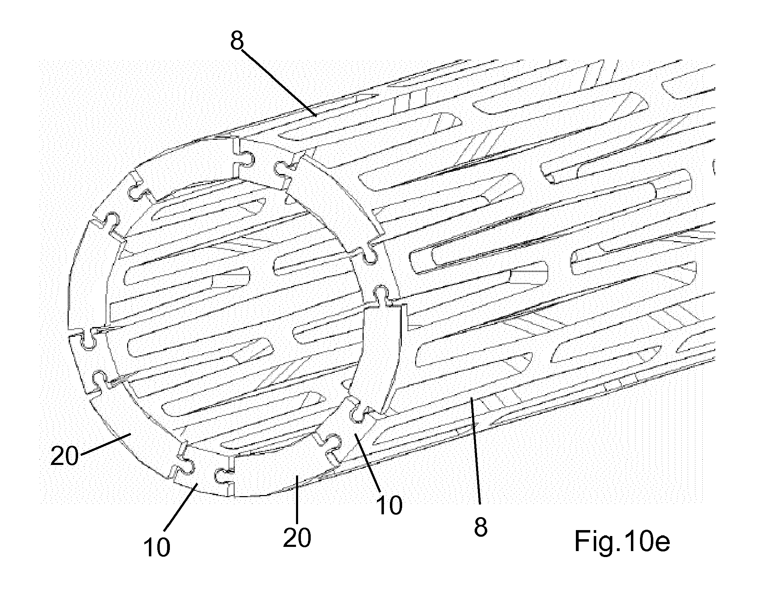

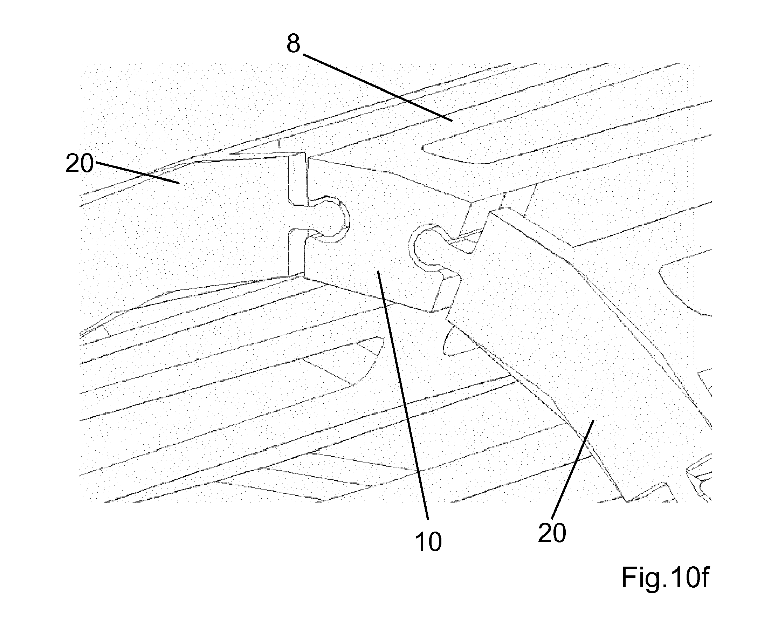

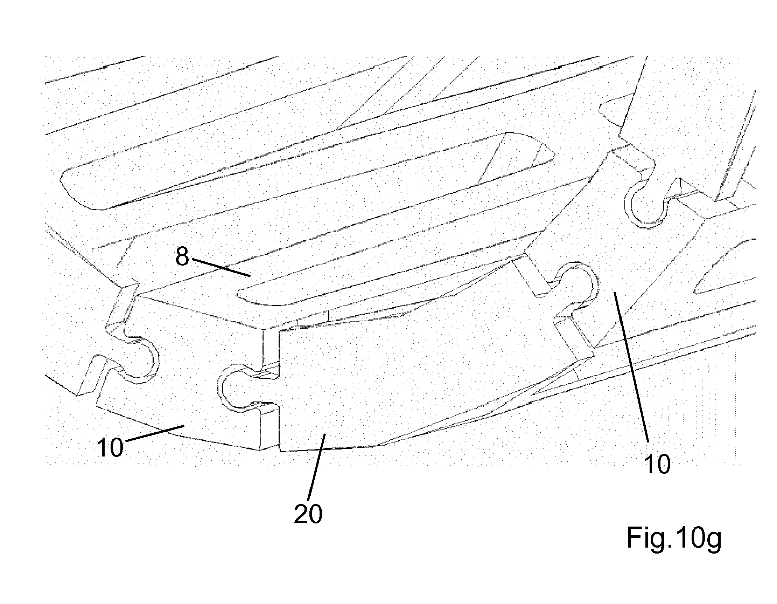

Bei diesem Ausführungsbeispiel weist jede Ausnehmung 11 jeweils eine seitlich (in Umfangsrichtung 6 verlaufende) Öffnung (Durchgang) 19 aus. Eine solche Öffnung 19 ist insbesondere in Fig. 4 gut zu erkennen. Die Öffnung 19 dient zum Einführen des Zapfens 21 in die Ausnehmung 11 während des Einrastens beim Übergang des Stents von dem expandierten Zustand (siehe Fig. 10a bis 10d) in den komprimierten Zustand (vgl. Fig. 10e bis 10g).

Die konkaven Wandabschnitte 15, 16 der Ausnehmung 11, die in radialer Richtung 5 liegen, erstrecken sich so weit in Richtung der Öffnung 19, dass sie mit ihren, am weitesten in Richtung der Öffnung 19 liegenden Spitzen Hinterschneidungen 17, 18 ausbilden. Die Hinterschneidungen 17, 18 bewirken, dass der Zapfen 21 auch bei einer während der Handhabung des Stents im komprimierten Zustand auftretenden Kraft in Umfangsrichtung 6, welche das erste Knotenelement 10 und das zweite Knotenelement 20 auseinander zieht, von der Ausnehmung 11 (bis zu einer bestimmten Maximalkraft) festgehalten wird bzw. in der Ausnehmung 11 eingerastet bleibt.

Die Höhe h der Zapfen 21 in radialer Richtung 5 ist deutlich kleiner als die Höhe H des zweiten Knotenelements 20 in die gleiche Richtung. Hierdurch wird erreicht, dass die Zapfen 21 nicht nach außen von dem Stent ab- oder vorstehen. Eine Verletzungsgefahr besteht daher nicht.

Im Folgenden soll nun das Herstellungsverfahren für einen Stent nach dem ersten Ausführungsbeispiel beschrieben werden.

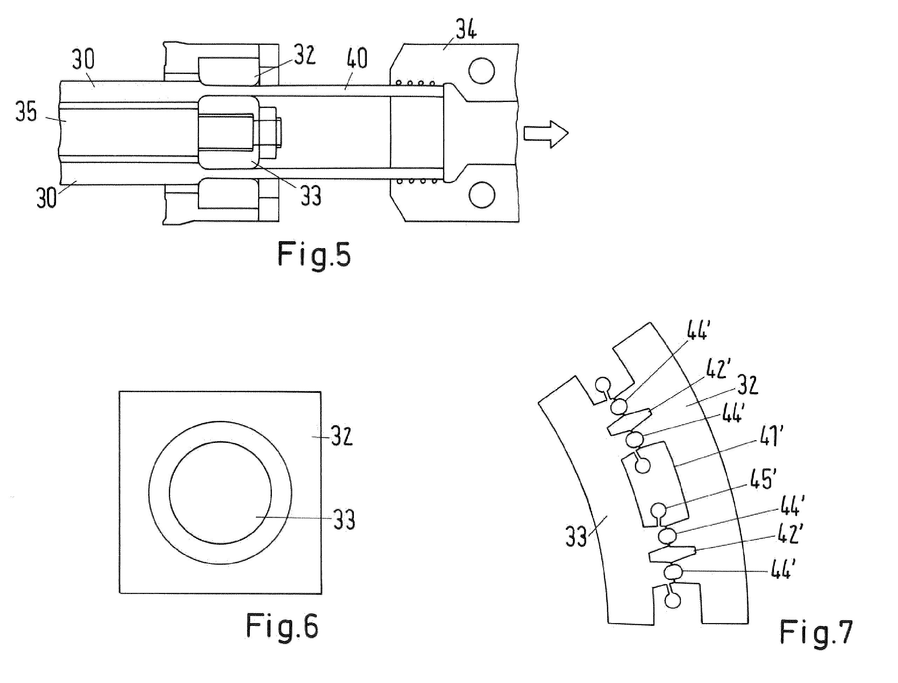

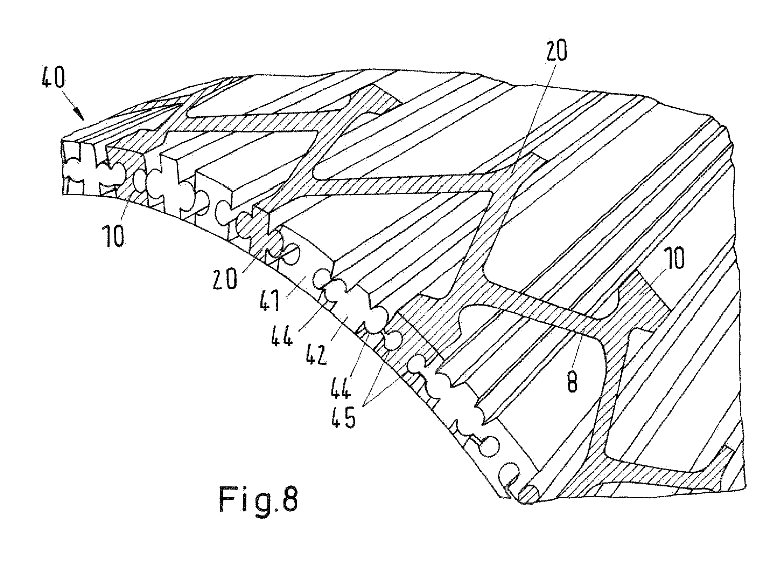

Zunächst wird mittels Gießen oder Sintern und ggf. nachträglichem Kernlochbohren ein Ausgangsrohr 30, beispielsweise aus Nitinol (alternativ bestehend aus Edelstahl, Titan, einer Titanlegierung, einer Kobalt-Chrom-Legierung, einer Nickelbasislegierung, einer Kupferlegierung, einer Magnesiumlegierung) hergestellt. Dieses Ausgangsrohr wird dann mittels Strangpressen, das in Fig. 5 dargestellt ist, zu einem Profilrohr 40, das in den Fig. 2 und 8 gezeigt ist, umgeformt. Hierfür wird das Ausgangsrohr 30 in ein Werkzeug bestehend aus einer Matrize 32 und einem Stopfen oder Dorn 33 eingeführt und vorzugsweise bei erhöhter Temperatur, z.B. bei einer Temperatur bis maximal 200°C, mittels einer Ziehzange 34, in die das vordere Ende des Profilrohrs 40 eingespannt ist, hindurch gezogen, wobei weiter bevorzugt die Matrize 32 vor der Umformung zur Verbesserung der Gleiteigenschaften erwärmt wird. Während des Umformschritts wird besonders bevorzugt ein Schmiermittel (lubricant) verwendet. Der Stopfen 33 ist dabei an einer Ziehstange 35 befestigt. Durch das Strangpressen wird in das Ausgangsrohr 30 ein Profil eingebracht, das eine gegenüber dem Ausgangsrohr 30 verringerte Wandstärke aufweist.

Die konzentrische Anordnung von Matrize 32 und Stopfen 33 ist in Fig. 6 lediglich schematisch dargestellt. Die Profilierung von Matrize 32 und Stopfen 33 wird in dieser Darstellung nicht gezeigt. Fig. 7 ist zu entnehmen, dass die Außenseite des Stopfens 33 und die Innenfläche der Matrize 32 zusammen ein komplementäres Profil, jeweils mit Ausnehmungen 41', 42' und 44' ausbilden, das zur Ausformung von in Längsrichtung verlaufenden Stegen 41, 42 und jeweils dazwischen liegenden und in Längsrichtung verlaufenden Zylindern 44 dient. Ferner weist der Dorn 33 Zapfen 45' auf, welche im Profilrohr 40 im Wesentlichen zylinderförmige, ebenfalls in Längsrichtung verlaufende Ausnehmungen 45 erzeugen.

Das durch Matrize 32 und Stopfen 33 geformte Profilrohr 40 setzt sich demnach zusammen aus in Längsrichtung verlaufenden Stegen 41, 42, welche abwechselnd angeordnet und jeweils über einen Zylinder 44 miteinander verbunden sind. Der erste Steg 41 weist zwei sich ebenfalls in Längsrichtung erstreckende, im Wesentlichen zylinderförmige Ausnehmungen 45 auf.

Das so hergestellte Profilrohr 40 wird im nächsten Schritt mittels Laserstrahlschneiden bearbeitet, um die Stege 8 und die Knotenelemente 10, 20 mit den Sperrelementen 11, 21 auszuschneiden. Die in radialer Richtung 5 verlaufenden Strahlen des Lasers sind in den Fig. 3 und 9 mit dem Bezugszeichen 50 versehen und zeigen in diesen Zeichnungen den Strahlverlauf im Bereich eines Knotenelements 10, 20. Die Laserstrahlen 50 entfernen im

Bereich der Stege 41 Material seitlich von den Ausnehmungen 45 derart, dass aus den Stegen 41 die ersten Knotenelemente 10 mit den Ausnehmungen 11 und seitlicher Öffnung 19 und aus den Stegen 42 die zweiten Knotenelemente 20 und aus den Zylindern 44 die Zapfen 21 entstehen.

Wie Fig. 8 zu entnehmen ist, werden die Laserstrahlen 50 zwischen den Knotenelementen 10, 20, die in Längsrichtung hintereinander liegen, derart geführt, dass aus der Struktur des Profilrohrs 40 nun schräg zur Längsrichtung die Stege 8 herausgeschnitten werden. Die Stege 8 laufen in Längsrichtung zu dem jeweils anderen Knotenelement 20, 10 zusammenlaufen ect.. Die Struktur, die aus einem Profilrohr 40 mittels Laserstrahl herausgeschnitten wird, ist in Fig. 8 schraffiert dargestellt.

Nach dem Laserstrahlschneiden wird der Stent ggf. noch Nachbehandlungsschritten wie Entgraten, Beizen und Elektropolieren unterzogen und wird dann fertiggestellt. Insbesondere werden in der Nachbehandlung ggf. durch das Laserstrahlschneiden entstandene Grate oder Unregelmäßigkeiten auf der Oberfläche des Stents entfernt. Der erfindungsgemäße Stent mit ersten Sperrelementen in Form von zylinderförmigen Ausnehmungen 11 und zweiten Sperrelementen in Form von Zapfen 21 kann danach auf den Ballon eines Katheter aufgecrimpt und hierdurch von dem expandierten Zustand (siehe Fig. 10a bis 10d) in den komprimierten Zustand (vgl. Fig. 10e bis 10g) überführt werden. Hierbei rasten die Zapfen 21 in die jeweils benachbarte Ausnehmung 11 ein und arretieren dadurch den erfindungsgemäßen Stent auch nach Abnahme des Crimpwerkzeugs im komprimierten Zustand.

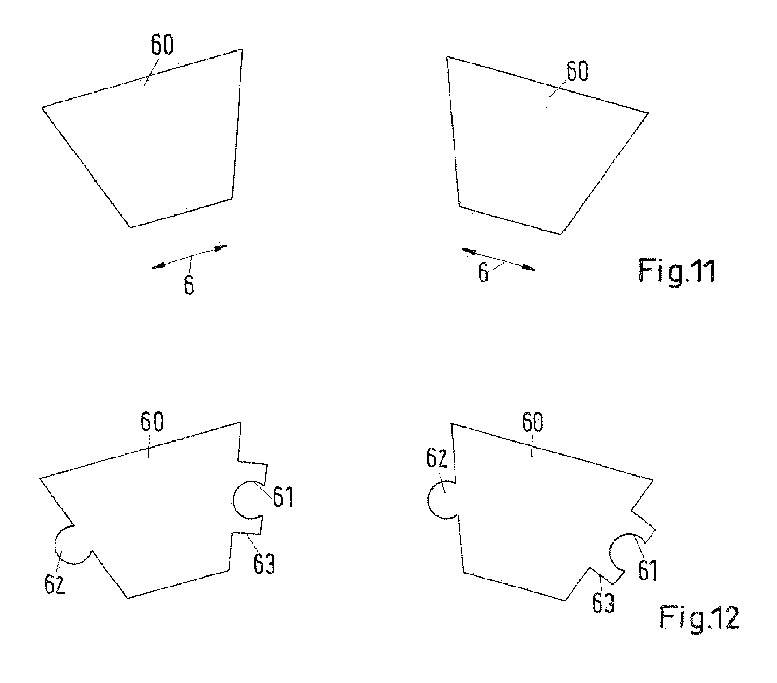

Die Fig. 11 bis 13 zeigen eine Abwandlung des ersten Ausführungsbeispiels eines erfindungsgemäßen Stents, welches an Knotenelementen 60 an der einen Seite ein erstes Sperrelement in Form einer zylinderförmigen Ausnehmung 61 und an der zweiten Seite ein zweites Sperrelement in Form eines zylinderförmigen Zapfens 62, welche sich jeweils in Umfangsrichtung 6 erstrecken, aufweisen. Hierbei sind die Ausnehmung 61 und der Zapfen 62 an benachbarten Knotenelementen 60 jeweils derart angeordnet, dass sie sich, wie in Fig. 12 gezeigt, gegenüber liegen.

Im Vergleich zum ersten Ausführungsbeispiel sind an jedem Knotenelement 60 des zweiten Ausführungsbeispiels zwei verschiedenartige Sperrelemente 61, 62 angeordnet, während beim ersten Ausführungsbeispiel an jedem Knotenelement 10, 20 zwei gleichartige Sperrelemente (entweder zwei Ausnehmungen 11 oder zwei Zapfen 21) vorgesehen sind. Alternativ können natürlich auch beim ersten Ausführungsbeispiel an jedem Knotenelement verschiedenartige Sperrelemente oder beim zweiten Ausführungsbeispiel an jedem Knotenelement gleichartige Sperrelemente angeordnet sein.

Die Ausnehmung 61 des zweiten Ausführungsbeispiels ist analog zu der Ausnehmung 11 des in den Fig. 1 bis 10 dargestellten Ausführungsbeispiels aufgebaut. Insbesondere weist die Ausnehmung 61 auch Hinterschneidungen neben einer Einführöffnung für den Zapfen 62 auf. Allerdings ragt das erste Sperrelement 61 in einen im Wesentlichen quaderförmigen Vorsprung oder Absatz 63 aus dem jeweiligen Knotenelement 60 heraus, während das erste Sperrelement 11 des ersten Ausführungsbeispiels innerhalb des Knotens 10, ohne separaten Absatz, angeordnet ist.

Der Zapfen 62 entspricht im Aufbau dem Zapfen 21 des in den Fig. 1 bis 10 dargestellten ersten Ausführungsbeispiels.

Das in den Fig. 11 bis 13 dargestellte zweite Ausführungsbeispiel kann hergestellt werden, indem ein analog zum ersten Ausführungsbeispiel hergestelltes Ausgangsrohr aus einem Material analog zum ersten Ausführungsbeispiel mittels Laserstrahlschneiden bearbeitet wird. Hierbei schneidet der Laserstrahl lediglich die Grundstruktur aus dem Ausgangsrohr bestehend aus den Stegen 8 und den Knoten 60 heraus. Der Umformschritt entfällt.

Nun wird auf die so hergestellte Stentstruktur ein Werkstoff , z.B. Nitinol, in einer dünnen Schicht in Pulverform aufgebracht.

Für die Herstellung des Pulvers wird zunächst das Vollmaterial verdüst, mechanisch zerkleinert oder in Lösung chemisch abgeschieden. Die für das Laserschmelzen verwendeten Werkstoffe sind Serienwerkstoffe, die keine Bindemittel enthalten. Die Werkstoffe umfassen beispielsweise Edelstahl, Werkzeugstahl, Aluminium, Aluminiumlegierungen, Titan, Titanlegierungen, Kobalt-Chrom, Nickelbasislegierungen, Kupferlegierungen, Keramik, Kunststoffe.

Dieser pulverförmige Werkstoff wird mittels eines Laser- oder Elektronenstrahls im Bereich der Sperrelemente 61, 62 lokal vollständig aufgeschmolzen und erstarrt dann wieder. Er bildet nach der Erstarrung eine feste Materialschicht, nämlich die Sperrelemente 61, 62. Die Herstellung der Sperrelemente 61, 62 kann dabei in mehreren Schritten erfolgen, wobei in jedem Schritt eine Schicht des jeweiligen Sperrelements 61, 62 fertiggestellt wird.

Für das Verfahren des selektiven Laserschmelzens oder selektiven Lasersinterns oder Rapid Prototyping werden die Daten für die Führung des Laserstrahls aus einem 3D-CAD-Körper mittels Software erzeugt.

Durch selektives Laserschmelzen gefertigte Elemente zeichnen sich durch eine große Dichte aus. Dies gewährleistet, dass die mechanischen Eigenschaften des generativ hergestellten Elements weitgehend denen des Grundwerkstoffs entsprechen.

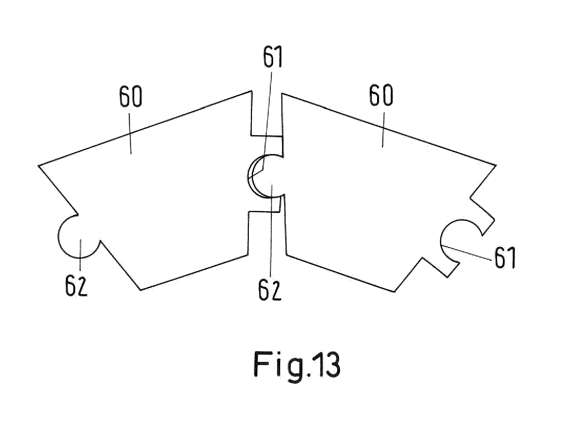

Anschließend wird das fertige Bauteil von überschüssigem Pulver gereinigt, und beispielsweise mittels Nachbehandlungsschritten wie Entgraten, Beizen und Elektropolieren fertiggestellt sowie ggf. analog zum ersten Ausführungsbeispiel auf den Ballon eines Katheters aufgecrimpt. Beim Crimpen rasten die Zapfen 62, wie in Fig. 13 dargestellt, in die Ausnehmungen 61 ein.

Alternativ können die Sperrelemente auch beispielsweise von einem Schweißdraht geformt und auf die Knotenelemente 60 aufgeschweißt oder aufgeklebt werden.

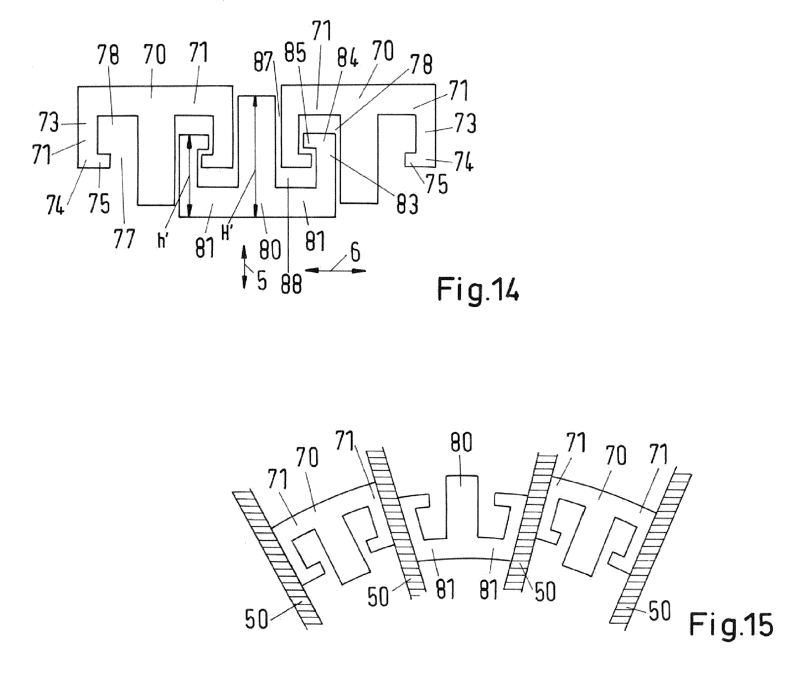

Schließlich zeigen die Fig. 14 und 15 ein drittes Ausführungsbeispiel eines erfindungsgemäßen Implantats. Hier werden an den Stegenden Knotenelemente 70, 80 ausgebildet, wobei das erste Knotenelement 70 jeweils zwei erste Sperrelemente 71 und das zweite Knotenelement 80 jeweils zwei zweites Sperrelemente 81 jeweils in Umfangsrichtung an beiden Seiten aufweisen.

Das erste Sperrelement 71 beinhaltet eine in U-Form ausgebildete Steganordnung mit einer in radialer Richtung 5 vorgesehenen Öffnung 77. Die Steganordnung umgibt eine im Querschnitt rechteckige Ausnehmung 78. Ein Schenkel des U wird dabei durch das Knotenelement 70 und der andere Schenkel des U durch einen in radialer Richtung 5 verlaufenden Steg 73 gebildet. An dem vorderen, in Bezug auf den Stent nach innen zeigenden Ende 74 des Stegs 73 ist in Umfangsrichtung ein weiteres Stegelement 75 angeordnet, das sich in Umfangsrichtung 6 erstreckt, jedoch die Öffnung 77 des U in radialer Richtung 5 noch offen lässt. Das Stegelement 75 bildet eine Hinterschneidung aus.

Das zweite Sperrelement 81 ist zu dem ersten Sperrelement 71 komplementär aufgebaut. Auch das zweite Sperrelement 81 bildet eine U-Form mit einer sich in radialer Richtung 5 erstreckenden Öffnung 87, die jedoch entgegengesetzt zu der Öffnung 77 des ersten Sperrelements 71 gerichtet ist, und einer durch das U umgebenen Ausnehmung 88. Ferner ist an dem vorderen Ende 84 eines seitlichen Steges 83 ein weiteres, sich in Umfangsrichtung 6 erstreckendes Stegelement 85 angeordnet, welches jedoch die Öffnung 87 nur teilweise verschließt und eine Hinterschneidung ausbildet.

Beim Crimpen verrastet das erste Sperrelement 71 mit dem zweiten Sperrelemente 81 wie in Fig. 14 gezeigt. Insbesondere wird das vordere Ende 74 mit dem quer verlaufenden Stegelement 75 des ersten Sperrelements 71 durch die Ausnehmung 88 des zweiten Sperrelements 81 aufgenommen und umgekehrt das vordere Ende 84 des Steges 83 mit dem quer verlaufenden Stegelement 85 durch die Ausnehmung 78 des ersten Sperrelements 71. Treten nun durch die Bewegung des Stents im komprimierten Zustand Kräfte in Umfangsrichtung 6 auf, so werden diese durch die Knotenelemente 70, 80 und die Stege 73, 83 der Sperrelemente 71, 81 aufgenommen. Bei Kräften in radialer Richtung 5 werden diese durch die die Hinterschneidungen ausbildenden Stegelemente 75, 85 bzw. die zwischen dem Steg 73 und dem Knotenelement 70 bzw. dem Steg 83 und dem Knotenelement 80 liegenden Mittelteile der U-Form der Sperrelemente 71, 81 aufgenommen.

Analog zum ersten Ausführungsbeispiel ist die Ausdehnung h' des zweiten Sperrelements 81 kleiner als die Ausdehnung H' des benachbarten zweiten Knotenelements 80, jeweils in radialer Richtung 5, so dass Verletzungen vermieden werden.

Fig. 15 zeigt einen Schritt bei der Herstellung eines derartigen Implantats. Analog zu dem Vorgehen beim ersten Ausführungsbeispiel kann bei diesem Ausführungsbeispiel zunächst mittels Strangpressen aus einem Ausgangsrohr ein Profilrohr erzeugt werden, aus dem dann die benötigten Strukturen, insbesondere die Stege, Knotenelemente und Sperrelemente mittels Laserstrahlschneiden durch radial verlaufende Laserstrahlen 50 (siehe Fig. 15) herausgeschnitten werden. Hieran schließen sich ggf. eine Nachbearbeitung und das Aufcrimpen auf den Ballon eines Katheters analog zu den vorstehend beschriebenen Ausführungsbeispielen an.

Die oben geschilderten erfindungsgemäßen Lösungen zeichnen sich dadurch aus, dass die Expansionssperren bestehend aus einem ersten Sperrelement und einem zweiten Sperrelement nicht nur in der Umfangsebene des Implantats wirken, sondern auch in radialer Richtung. Hierdurch kann der komprimierte Zustand auch bei verschiedensten Bewegungen, die ein solches Implantat beispielsweise beim Einführen in den Körper oder beim Durchführen durch ein Gefäß vollführt, sicherer beibehalten werden. Die Expansionssperren befinden sich über die Stentlänge und den Umfang des Implantats verteilt. Die Expansionssperren rasten während des Crimpens ein und verhindern so die selbständige Expansion des Stents. Durch die mittels Dilatation des Ballons aufgebrachten höheren Kräfte können die Verrastungen der Expansionssperren wieder gelöst werden, so dass das Implantat dann am Ort der Behandlung selbständig oder aufgrund der Dilatationskräfte des Ballons expandieren kann.

### Bezugszeichenliste

- 5: radiale Richtung
- 6: Umfangsrichtung
- 8: Steg
- 10: erstes Knotenelement
- 11: Ausnehmung
- 14, 15, 16: konkaver Abschnitt
- 17, 18: Hinterschneidung
- 19: Öffnung
- 20: zweites Knotenelement
- 21: Zapfen
- 23: Hals
- 24, 25, 26: konvexer Abschnitt
- 30: Ausgangsrohr
- 32: Matrize
- 33: Stopfen, Dorn
- 34: Ziehzange
- 35: Ziehstange
- 40: Profilrohr
- 41, 42: Steg
- 44: Zylinder
- 45: Ausnehmung
- 50: Laserstrahl
- 60: Knotenelement
- 61: Ausnehmung
- 62: Zapfen
- 63: Absatz
- 70: Knotenelement
- 71: erstes Sperrelement
- 73: Steg
- 74: vorderes Ende des Stegs 73

- 75: Stegelement
- 77: Öffnung

- 78: Ausnehmung
- 80: zweites Knotenelement
- 81: zweites Sperrelement
- 83: Steg
- 84: vorderes Ende des Stegs 83
- 85: Stegelement
- 87: Öffnung
- 88: Ausnehmung
- 41': Ausnehmung
- 42': Ausnehmung
- 44': Ausnehmung
- 45': Zapfen
- h: Höhe des Zapfens 21
- h': Höhe des Sperrelements 81
- H, H': Höhe des Knotenelements

## Patentansprüche

1. Implantat mit einer durchbrochenen, vorzugsweise hohlzylinderförmigen Grundstruktur zusammengesetzt aus einer Vielzahl von Stegen (8), von denen mindestens jeweils zwei an Knotenelementen (10, 20, 60, 70, 80) miteinander verbunden sind, wobei das Implantat einen expandierten Zustand und einen komprimierten Zustand einnimmt, **dadurch gekennzeichnet, dass** an einer Vielzahl von ersten Knotenelementen (10, 60, 70) jeweils mindestens ein erstes Sperrelement (11, 61, 71) und an einer Vielzahl von zweiten, zu jeweils einem ersten Knotenelement (10, 60, 70) benachbarten Knotenelementen (20, 60, 80) jeweils mindestens ein zweites Sperrelement (21, 62, 81) vorgesehen ist, wobei jeweils ein erstes Sperrelement (11, 61, 71) und ein benachbartes zweites Sperrelement (21, 62, 81) beim Übergang des Implantats von dem expandierten Zustand in den komprimierten Zustand, vorzugsweise beim Crimpen, miteinander verrasten und hierdurch eine Sperre gegen den Übergang zurück in den expandierten Zustand ausbilden, wobei die Sperre gegen eine Kraft in Umfangsrichtung (6) und gegen eine Kraft in radialer Richtung (5) sperrt.

2. Implantat nach Anspruch 1, **dadurch gekennzeichnet, dass** das erste Sperrelement als ein weibliches Element, welches in Umfangsrichtung (6) offen ist, mit einer Hinterschneidung (17, 18) ausgebildet ist, beispielsweise als eine zumindest abschnittsweise kugelförmige und/oder zylinderförmige Ausnehmung (11, 61), und dass das zweite Sperrelement als ein männliches Element ausgebildet ist, beispielsweise als ein zumindest abschnittsweise kugelförmiger und/oder zumindest abschnittsweise zylinderförmiger Zapfen (21, 62).

3. Implantat nach Anspruch 1, **dadurch gekennzeichnet, dass** das erste Sperrelement als ein im Querschnitt U-förmiges Element (71) mit einer ersten Öffnung (77) in eine erste radiale Richtung (5) sowie mit einer an der ersten Öffnung (77) angeordneten Hinterschneidung (75) und das zweite Sperrelement (81) ebenfalls als ein im Querschnitt U-förmiges Element mit einer zweiten Öffnung (87) in eine zweite, zur ersten radialen Richtung (5) entgegengesetzten Richtung sowie mit einer an der zweiten Öffnung (87) angeordneten Hinterschneidung (85) ausgebildet ist.

4. Implantat nach einem der vorhergehenden Ansprüche, **dadurch gekennzeichnet, dass** die Ausdehnung (h, h') des zweiten Sperrelements (21, 81) in radialer Richtung (5) kleiner ist als die Ausdehnung (H, H') des jeweils benachbarten zweiten Knotenelements (20, 80) in radialer Richtung (5).

5. Implantat nach einem der vorhergehenden Ansprüche, **dadurch gekennzeichnet, dass** das erste Sperrelement (10, 70) und das zweite Sperrelement (20, 80) aus jeweils einem entsprechend geformten Abschnitt eines sich parallel zur Längsachse des Implantats erstreckenden Profils ausgebildet sind.

6. Implantat nach Anspruch 5, **dadurch gekennzeichnet, dass** die Stege (8) aus Abschnitten von sich parallel zur Längsachse des Implants erstreckenden Profilen ausgebildet sind.

7. System aus einem Implantat nach einem der vorhergehenden Ansprüche und einem Katheter mit einem Ballon, wobei das Implantat auf den Ballon des Katheters aufgecrimpt ist.

8. Verfahren zur Herstellung eines Implantats, insbesondere nach einem der Ansprüche 1 bis 6, mit den folgenden Schritten:
- Herstellen eines Ausgangsrohrs (30), vorzugsweise mittels Gießen oder Sintern oder Rapid Prototyping und ggf. Kernlochbohren,
- Umformen des Ausgangsrohrs (30) zu einem Profilrohr (40) mittels Durchdrücken oder Durchziehen und unter Anwendung eines Werkzeugs umfassend eine Matrize (32) und einen Stopfen (33),
- Ausschneiden der Knotenelemente (10, 20, 70, 80) und der Stege (8) aus dem Profilrohr mittels Laserstrahlschneiden, wobei die Laserstrahlen (50) vorzugsweise im Wesentlichen in radialer Richtung verlaufen.

9. Verfahren zur Herstellung eines Implantats, insbesondere nach einem der Ansprüche 1 bis 6, mit den folgenden Schritten:
- Ausformen des Implantats mittels Rapid Prototyping.

10. Verfahren zur Herstellung eines Implantats, insbesondere nach einem der Ansprüche 1 bis 6, mit den folgenden Schritten:
- Herstellen eines Ausgangsrohrs, vorzugsweise mittels Gießen oder Sintern oder Rapid Prototyping und ggf. Kernlochbohren,
- Ausschneiden der Knotenelemente (60) und der Stege aus dem Ausgangsrohr mittels Laserstrahlschneiden,
- separates Herstellen einer Vielzahl von ersten Sperrelementen und einer Vielzahl von zweiten Sperrelementen und Anbringen der ersten Sperrelemente und der zweiten Sperrelemente abwechselnd an in Umfangsrichtung jeweils benachbarten Knoten, vorzugsweise mittels Kleben oder Schweißen oder Pulverspritzgießen oder Erodieren,
- Herstellen einer Vielzahl von ersten Sperrelementen (61) und einer Vielzahl von zweiten Sperrelementen (62) an den Knotenelementen (60) mittels selektivem Laserschmelzen.

11. Verfahren zur Herstellung eines Systems aus einem Katheter mit Ballon und einem Implantat, insbesondere nach Anspruch 7, wobei das Implantat nach einem der in den Ansprüchen 8 bis 10 angegebenen Verfahren hergestellt, danach auf dem Ballon des Katheters angeordnet und dann auf diesen aufgecrimpt wird.
